(19) Europäisches Patentamt
European Patent Office
Office européen des brevets

(11) **EP 4 230 114 A1**

(12) **EUROPEAN PATENT APPLICATION**

(43) Date of publication:
**23.08.2023 Bulletin 2023/34**

(51) International Patent Classification (IPC):
***A61B 5/00*** *(2006.01)*

(21) Application number: **22157370.2**

(52) Cooperative Patent Classification (CPC):
**A61B 5/6817; A61B 5/4809;** A61B 5/12;
A61B 5/486

(22) Date of filing: **17.02.2022**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **HIWALE, Sujitkumar**
**Eindhoven (NL)**
• **CHAKRABARTI, Biswaroop**
**Eindhoven (NL)**

(74) Representative: **Philips Intellectual Property & Standards**
**High Tech Campus 52**
**5656 AG Eindhoven (NL)**

(54) **A METHOD AND SYSTEM FOR DETECTING DROWSINESS AND/OR SLEEP**

(57)     A method and system is provided for detecting drowsiness and/or sleep and then for example controlling the volume of audio delivered from an in-ear speaker. A detected external ear pressure, between the ear drum and the in-ear speaker, is obtained and is processed together with the frequency and amplitude characteristics of the delivered audio. In this way, a baseline ear pressure over time is obtained. Drowsiness and/or sleep is detected from the derived baseline ear pressure, for example so that the volume of the delivered audio can be adapted accordingly.

Fig. 3

FIG. 2

**Description**

FIELD OF THE INVENTION

The invention relates to the detection of drowsiness and/or sleep. For example, such detection may be used to control sound delivered by an-in ear speaker, and in particular to provide automatic turning off when a listener falls asleep. It may also be used to control other external devices.

BACKGROUND OF THE INVENTION

Music has always been an integral part of modern human culture. Advancement in digital technology has made it possible to listen to music at any time anywhere using personal listening devices (PLDs) such as iPods, mobile phones, and tablet computers.

Music has been shown to have beneficial effects and has been used as a part of music therapy for management of many neurological and mental disorders. However, recent scientific studies have found that excessive listening to music on PLDs can induce hearing loss or accelerate age-related hearing loss. The reported prevalence of hearing loss with music exposure in young adults ranges from 9.3% to 14%.

It is further observed that approximately 58% of adolescents and college students exceed the recommended maximum noise exposure during listening to music. It has been observed that not only volume levels of music but also total duration of exposure significantly correlates with hearing loss. If not detected and treated in time, hearing loss from such exposure can become permanent.

PLDs are often used with in-ear speakers, i.e. earplugs/earbuds. Many people fall asleep while listening to music. A continuous and prolonged exposure to music in such situations could be harmful to the ears.

It has been propose to automatically turn down music volume when sleep is detected. For example, WO 2012058886 and CN 109561219 each disclose the use of pressure detection for detecting a pulse, and then identifying sleep from the pulse characteristics such as the heart rate variability.

However, the detection of the pulse may not be accurate, because the heart pulse is for example drowned out by the music being listened to, which may even have a rhythm which corresponds to the pulse rate.

SUMMARY OF THE INVENTION

The invention is defined by the claims.

According to examples in accordance with an aspect of the invention, there is provided a method of detecting drowsiness and/or sleep, comprising:

receiving a detected external ear pressure, between the ear drum and an in-ear speaker, from a pressure sensor forming part of the in-ear speaker during the delivery of audio to the in-ear speaker;
processing the frequency and amplitude characteristics of the delivered audio and the external ear pressure to derive a baseline ear pressure over time; and
detecting drowsiness and/or sleep from the derived baseline ear pressure.

This method uses pressure sensing to detect drowsiness and/or sleep. Instead of using pressure sensing to detect a pulse in known manner, a baseline (residual) pressure is obtained by separating the pressure resulting from the physiology of the subject and the pressure created by the audio. The baseline pressure is the pressure resulting from the physiology of the subject after removal of the pressure effects of the music (or other audio) being listened to. The baseline pressure enables detection of various events, such as short term pressure changes due to yawning, or gradual pressure changes over time.

The detected drowsiness and/or sleep may be used to control devices, for example to turn them off or reduce the volume. For example, in response to the detected drowsiness or sleep, the volume delivered by the in-ear speaker itself may be lowered or the audio may be switched off (i.e. the volume is adapted to zero). The volume may be decreased progressively to zero.

At its simplest level, the method may be implemented without the need for any dedicated external sensor to detect the sleep or drowsiness state. Instead, the basic hardware present in the in-ear speaker itself is used for detection of sleep. The acoustic transducer in the earbuds can be reversed to act as a microphone to detect the pressure changes. For this purpose, a duty cycle control may be used to alternate between the speaker function and the pressure sensor (i.e. microphone) function.

The method for example includes applying timestamps to the audio and to the detected ear pressure using a shared clock, so that the different signals can be synchronized for analysis.

Detecting drowsiness and/or sleep may comprise:

detecting yawning events from the baseline ear pressure; and/or
detecting an increase in middle ear pressure associated with sleep from the baseline ear pressure.

Yawing signifies the onset of sleep. A frequency of yawning events may for example be monitored over time. The middle ear pressure is known to increase during sleep, and by separating the audio from the baseline pressure, this may also be detected from the measured external ear pressure.

The method may further comprise detecting the correct fitting of the in-ear speaker to the ear, and generating an output signal to indicate if there is incorrect fitting.

The pressure sensing requires a closed cavity to be formed to enable correct measurement and interpretation of the baseline pressure measurements. Thus, if there is incorrect fitting, a warning may be provided and/or the control based on sleep detection may be disabled.

The method may further comprise detecting an external ear pressure during a calibration phase to determine subject-specific pressure characteristics.

This enables the analysis of the baseline pressure to be more accurate for the particular subject. The calibration phase for example involves the subject performing tasks such as (simulated) yawning, swallowing, and/or jaw movements. This calibration phase may also be used to detect ear pathologies.

Deriving a baseline ear pressure over time may comprise using an analytical model of the ear canal. This model takes account of the ear canal chamber volume and shape.

The analytical model is for example adapted to the subject by sensing one more ear canal dimension or shape characteristics.

Proximity sensors or ultrasound sensors may be used for this purpose. The model may also take account of the age and gender of the subject, for example.

The method may further comprise sensing movement of the subject using an inertia monitoring unit, and detecting drowsiness and/or sleep additionally from the sensed movement.

Head movement is thereby monitored during listening to audio. It is expected that when a person becomes drowsy their head movements will decrease and eventually the head will become stable in a particular position for some time. The head movements then can be correlated with the baseline pressure changes to detect drowsiness or sleep. The inertia monitoring unit may form part of the in-ear speaker.

The invention also provides a method of controlling a device, comprising:

detecting drowsiness and/or sleep using the method as defined above; and
controlling the device in dependence on the detection of drowsiness and/or sleep.

The device being controlled may be the in-ear speaker itself (e.g. by controlling a sound source which delivers the audio to the in-ear speaker), but it may be an external device such as a television, or even a non-audio device such as lighting, heating, putting mobile phone in "sleep mode" etc.

Thus, in one example, the device comprises the in-ear speaker, and controlling the device comprises adapting the volume of the delivered audio (or the on-off status) in dependence on the detection of drowsiness and/or sleep.

The method may further comprise detecting arousal from sleep and controlling the delivery of audio by the in-ear speaker to improve sleep quality. Arousal from sleep may be detected by other sensors, such as an inertia monitoring unit of a mobile phone, or a EEG sensor. When arousal is detected, other devices may be controlled, for example a coffee machine.

The method may further comprise controlling a valve to vent the space between the ear drum and the in-ear speaker to the ambient surroundings, in dependence on the detection of drowsiness and/or sleep.

The valve provides a physical connection which can be opened to create an air passage between the external ear canal and the outside so that the canal is no longer a closed chamber. This connection may be opened when turning the audio volume down or off. This will reduce humidity in the ear canal and establish a near normal airflow, thus reducing a risk of infection.

The invention also provides a computer program comprising computer program code which is adapted, when said program is run on a computer, to implement the method defined above.

The invention also provides drowsiness and/or sleep detection system, comprising:

an in-ear speaker having a pressure sensor for detecting an external ear pressure between the ear drum and the in-ear speaker; and
a processor, wherein the processor is adapted to:

receive a sensed external ear pressure from the pressure sensor;
process frequency and amplitude characteristics of audio that is delivered to the in-ear speaker and the corresponding sensed external ear pressure, thereby to derive a baseline ear pressure overtime; and
detect drowsiness and/or sleep from the derived baseline ear pressure.

The processor is for example adapted to detect drowsiness and/or sleep by:

detecting yawning events from the baseline ear pressure; and/or
detecting an increase in middle ear pressure associated with sleep from the baseline ear pressure.

The invention also provides an audio delivery system comprising the drowsiness and/or sleep detection system defined above, wherein the processor is adapted to adapt the volume (or on-off status) of the delivered audio in dependence on the detection of drowsiness and/or sleep.

The system (for drowsiness detection or for audio delivery) may further comprise a sensor arrangement for detecting the correct fitting of the in-ear speaker to the ear, wherein the processor is adapted to generate an output signal to indicate if there is incorrect fitting.

The system (for drowsiness detection or for audio delivery) may further comprise an inertia monitoring unit, wherein the processor is adapted to detect drowsiness and/or sleep additionally from the sensed movement.

The audio delivery system may further comprise a sensor arrangement for detecting arousal from sleep, wherein the processor is adapted to control the delivery of audio to improve sleep quality in response to detection of arousal from sleep.

The system (for drowsiness detection or for audio delivery) may also comprise a valve to vent the space between the ear drum and the in-ear speaker to the ambient surroundings, wherein the processor is adapted to control the valve in dependence on the detection of drowsiness and/or sleep.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows an audio delivery system;
Figure 2 shows a calibration phase of a method of controlling the volume of audio delivered from an in-ear speaker;
Figure 3 shows the volume control method after the calibration phase; and
Figure 4 shows a plot of the baseline pressure over time and shows a single yawning event.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for detecting drowsiness and/or sleep and then for example controlling the volume of audio delivered from an in-ear speaker. A detected external ear pressure, between the ear drum and the in-ear speaker, is obtained and is processed together with the frequency and amplitude characteristics of the delivered audio. In this way, a baseline ear pressure over time is obtained. Drowsiness and/or sleep is detected from the derived baseline ear pressure, for example so that the volume of the delivered audio can be adapted accordingly.

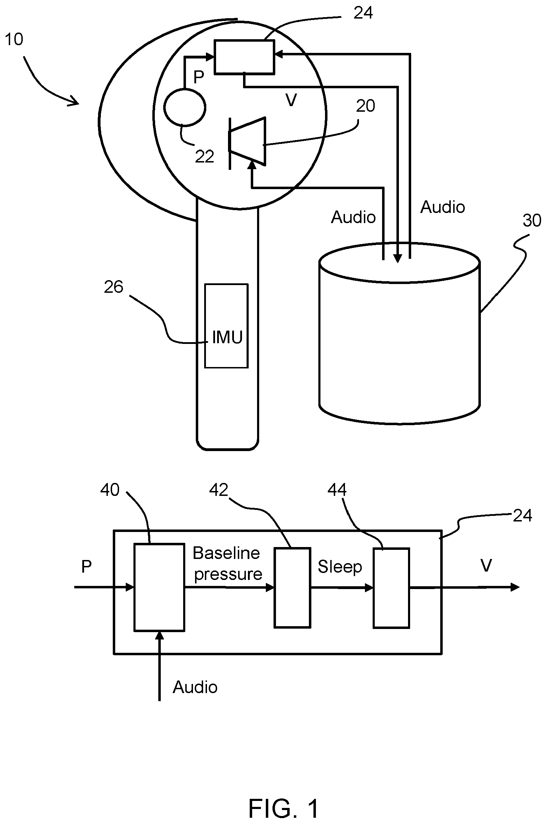

Figure 1 shows an audio delivery system 10 comprising an in-ear speaker 20 having a pressure sensor 22 for detecting an external ear pressure between the ear drum and the in-ear speaker 20.

Audio is delivered to the system 10 from a sound source 30. Typically, the sound source 30 is a mobile phone or music player, and the communication between the sound source and the system 10 is via short range wireless communication such as Bluetooth™. Of course, a wired connection is also possible.

A processor 24 of the system receives the sensed external ear pressure from the pressure sensor 22 and also analyzes the frequency and amplitude characteristics of the audio that is delivered to the in-ear speaker. Optionally, the system also has an inertia monitoring unit, IMU, 26.

The functions of the processor are shown in Figure 1.

A first module 40 receives the sensed external ear pressure from the pressure sensor 22 and receives the audio data. A second module 42 extracts from the sensed external ear pressure those components which derive from the audio itself. In this way, a baseline ear pressure over time is derived. This baseline ear pressure is derives from the physiology of the listener.

A second module 42 processes the baseline ear pressure to detect drowsiness and/or sleep and a third module 44 of the processor 24 then generates a volume control signal V to adapt the volume of the delivered audio in dependence on the detection of drowsiness and/or sleep. The volume control signal V is communicated back to the sound source 30.

Figure 1 shows a single in-ear speaker, but of course the system will typically comprise a pair of in-ear speaker. The sleep detection may be implemented in one or both in-ear speakers.

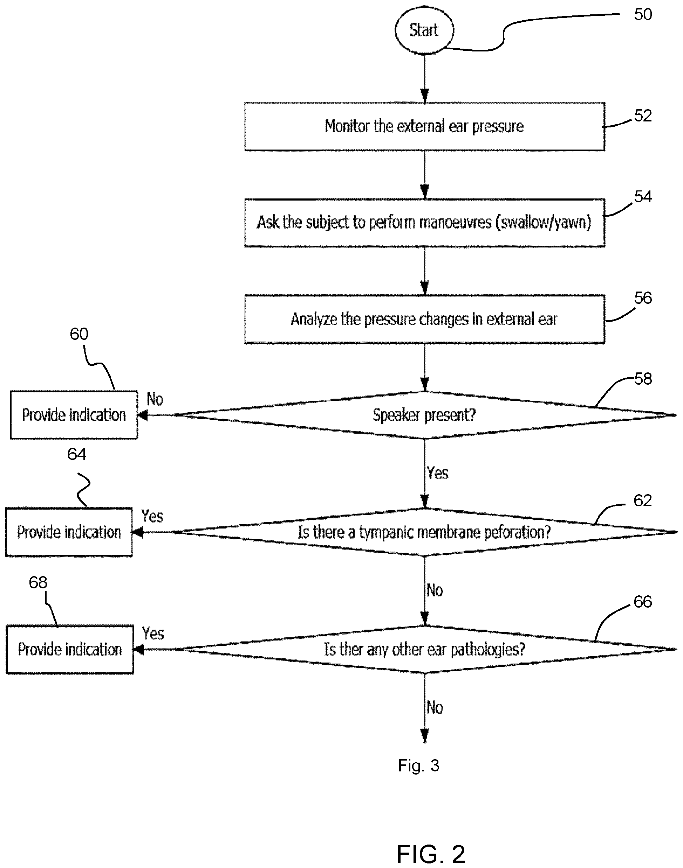

Figure 2 shows the first steps of a method of controlling the volume of audio delivered from an in-ear speaker. The steps of Figure 2 may be considered to be part of a calibration phase, before the user of the system uses the speaker to listen to audio.

In use, the in-ear speaker or speakers are inserted in the external ear in step 50 such that they seal the ear canal from the external atmosphere. This essentially creates a closed chamber. The pressure sensor 22 is on one side of the chamber and the tympanic membrane is on the other side.

The external ear pressure is detected and monitored over time in step 52 while the subject is asked in step 54 to perform some simple manoeuvers such as swallowing, yawning and jaw drops. These actions create pressure changes in the external ear and they are analyzed in step 56. This information is used as a baseline for subsequent processing.

This information is also used in step 58 to detect that the in-ear speaker is correctly positioned and functioning, and able to measure the baseline pressure in the ear canal. If the in-ear speaker is not working properly this can be indicated using an audio-visual indicator in step 60.

Existing pathologies in the external ear may also be examined by analyzing pressure changes in the external ear. If any existing pathologies are present this can again be indicated by means of audio-visual indicators.

For example, detection of tympanic membrane perforation is carried out in step 62 and if present this is indicated by means of audio-visual indicators in step 64.

Detection of other pathologies is carried out in step 66 and if present this is indicated by means of audio-visual indicators in step 68.

The calibration steps above are optional. The calibration to adapt the system to an individual subject is preferred, but the detection of pathologies is entirely optional.

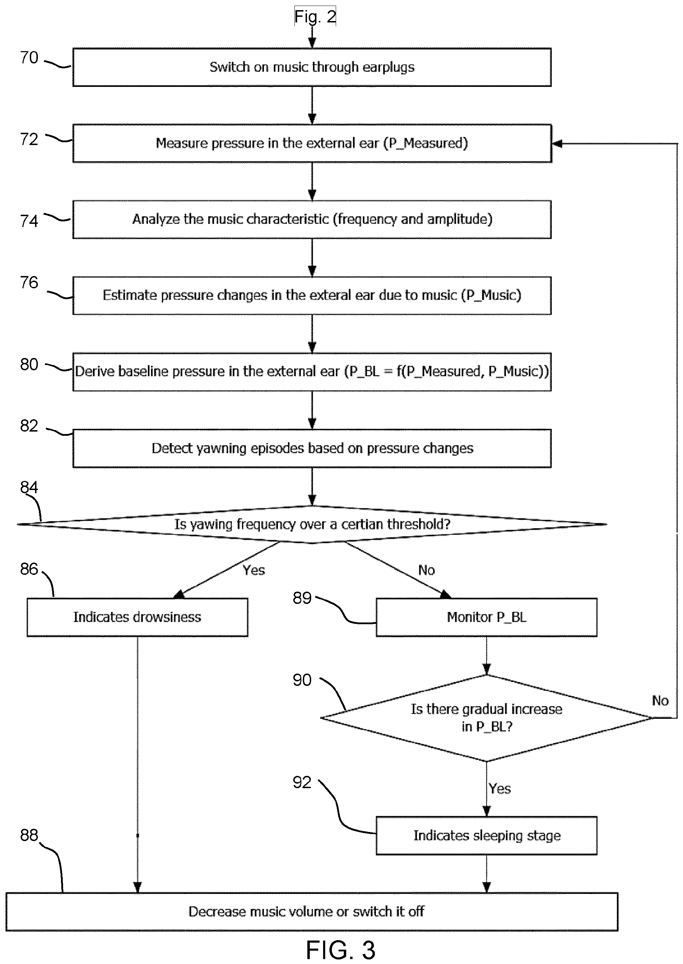

Figure 3 shows the volume control method after the calibration phase.

The user turns on the audio in step 70. This automatically activates the pressure senor so that the pressure is measured in step 72. The pressure in the external ear is monitored in real-time and is denoted P_Measured.

The played audio generates sound waves which travel through air by displacing nearby air columns. In the external ear canal, which is sealed at one end by the in-ear speaker and by the tympanic membrane at the other end, this results in change in pressure in the ear canal.

The change in pressure in external ear canal will depend on both the frequency and amplitude of the sound waves resulting from the played audio, and the physical dimensions of external ear canal. The external ear canal is roughly an elliptical cylinder and is about 25 mm long with a diameter of about 7 mm. To measure pressure changes, a lossless cylindrical-tube model has been proposed as an approximation for the extremal ear canal. The volume of the closed chamber created by the in-ear speakers thus can be calculated as follows,

$$V = \pi r 2 h \qquad (1)$$

where, h is a distance between the pressure sensor and the eardrum and r is the radius of the ear canal. Standard values may be used for h and r, for example based on age and gender. However, exact values may also be used by incorporating using suitable sensors such as proximity sensors, ultrasound sensors etc.

The audio characteristics, in particular frequency and amplitude are analyzed by the processor in step 74. Using the anatomical model of external ear as explained above, the changes in external ear pressure for the particular subject using the system can be derived for a given frequency and amplitude of the played audio. The pressure resulting from the audio is denoted as P_Music and is obtained in step 76 using the frequency and amplitude information for the audio.

The processor receives the pressure measurements P_Measured as well as the audio that is being played. Timestamps are for example used so that the pressure waveform over time can be aligned with the audio data that was playing, and that resulted in the pressure waveform.

The measured pressure is dependent on both a baseline pressure (P_BL) in the ear canal and pressure changes due to audio (P_Music). This can be expressed as,

$$P\_Measured = f(P\_BL, P\_Music) \qquad (2)$$

Based on empirical or experimental data it is possible to derive a mathematical model to derive the baseline external ear pressure (P_BL) from the measured pressure P_Measured in the presence of audio in the external ear (with corresponding pressure PMusic).

The baseline external ear pressure can thus be obtained in step 80 based on the mathematical model, which can be expressed as:

$$P\_BL = f(P\_Measured, P\_Music) \qquad (3)$$

The baseline external ear pressure is for example obtained using a machine learning algorithm. Experimental data is used for the training, and standard machine learning tools are used, such as a linear regression, to derive the relationship of Equation (3).

For a linear regression model, this can be written as:

$$P\_BL = \beta0 + \beta1\ (P\_Measured) + \beta2(P\_Music),$$

where $\beta1$ is a constant term and $\beta1$ and $\beta2$ are coefficients derived using experimental data.

The baseline external ear pressure is used to detect drowsiness and/or sleep.

The state of drowsiness before sleep is marked by frequent yawning. During yawning, pressure in the nasopharyngeal cavity increases. The middle ear is connected to nasopharynx via the Eustachian tube. Changes in the middle ear pressure result in tympanic membrane displacement (bulging or retraction). In particular, an increase in the middle ear pressure during yawning results in a transient bulging of tympanic membrane toward the external side.

As a result, yawning episodes can be detected in step 82 based on pressure changes in the external ear baseline pressure. Measurements on a sufficiently large sample population can be used to create a database of expected pressure changes in the external ear during yawning (in the presence of audio). Every episode of yawning, as detected based on the pressure signal characteristics, is timestamped for further processing. In step 84 it is detected if a frequency of yawning increases (over a predefined threshold) for a predefined period. This analysis can be used to indicate a state of drowsiness in step 86.

This information is then used to either lower the audio volume or switch off the audio completely in step 88.

Instead of, or as well as, detecting drowsiness based on yawning, the pressure monitoring may also be used to detect sleep. It is known that during sleep there is a slow increase in middle ear pressure. The increase in middle ear pressure will lead to bulging of tympanic membrane outwards. This will lead to a slow increase in baseline pressure in the external ear if it forms a closed cavity. Thus, in step 89 the baseline pressure P_BL is monitored and in step 90 it is determined if there is a detected gradual increase in this baseline external ear pressure. If there is, this is used in step 92 to determine that an individual has fallen asleep so that again the audio volume is lowered or the audio is switched off completely in step 88.

If neither drowsiness nor sleep are detected, the method returns to step 72 for a next analysis.

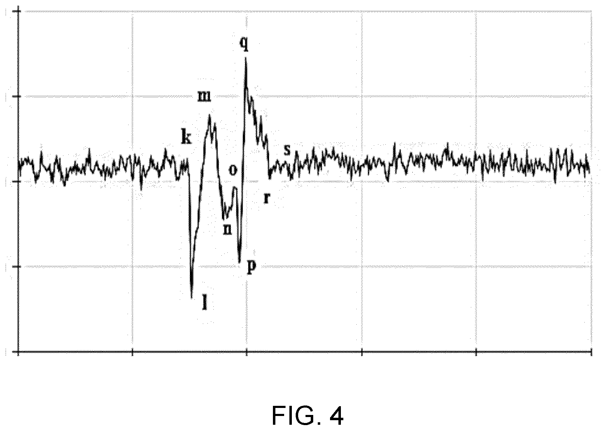

Figure 4 shows a plot of the baseline pressure over time and shows a single yawning event. There are various characteristic features (labeled k to s) which can be used to enable reliable detection of yawning from changes in pressure.

The in-ear speaker may additionally comprise an inertia measuring unit (IMU) to monitor head movements during listening to audio. It is expected that when a person becomes drowsy his head movements will decrease and eventually the head will become stable in a particular position for a prolonged period time. The head movements then thus be correlated with pressure changes in the external ear to detect drowsiness or sleep and control the audio accordingly.

Additional sensing may take place for example using sensors already present in the audio source, e.g. mobile phone. A mobile phone will include an IMU and other sensors, such as electroencephalography (EEG) sensors, may be used for sensing drowsiness or sleep.

The system may also detect arousal from sleep, for example based on pressure change and IMU measurement patterns. The system may then play a suitable masking noise (e.g. a white or brown or pink noise of predetermined intensity) in case of detected impending arousal to improve sleep quality

In another example, the in-ear speaker may include a valve to vent the space between the ear drum and the in-ear speaker to the ambient surroundings. Opening the valve means the canal is no longer a closed chamber. The system can then be configured to open this connection when turning the audio volume down based on drowsiness detection. This will help reducing humidity in the ear canal and establishing near normal airflow, thus reducing the risk of infections.

The example above shows a pressure sensor as a separate added component in the in-ear speaker. However, the speaker may itself be used as a pressure sensor (i.e. functioning as a microphone), for example with duty cycle control to alternate between the pressure sensing and speaker functions.

The main example of interest is to control the audio delivered by the in-ear speaker. However, other devices may be controlled based on the detection of sleep or drowsiness, such as lighting, heating, or other sound sources such as a television or external audio device. The control may comprise level control (e.g. volume or lighting level) or on-off control. Thus, more generally, the invention provides a method of detecting drowsiness or sleep using the pressure chamber formed by an in-ear speaker, and this information may then be used in any desired manner, to control external devices and/or the audio delivery by the in-ear speaker itself.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.


**Claims**

1. A method of detecting drowsiness and/or sleep, comprising:

    (72) receiving a detected external ear pressure, between the ear drum and an in-ear speaker, from a pressure sensor forming part of the in-ear speaker during the delivery of audio to the in-ear speaker;
    (80) processing the frequency and amplitude characteristics of the delivered audio and the external ear pressure to derive a baseline ear pressure over time; and
    (86; 92) detecting drowsiness and/or sleep from the derived baseline ear pressure.

2. The method of claim 1, comprising:

    (82) detecting yawning events from the baseline ear pressure; and/or
    (90) detecting an increase in middle ear pressure associated with sleep from the baseline ear pressure.

3. The method of any one of claims 1 to 2, further comprising (58) detecting the correct fitting of the in-ear speaker to the ear, and (60) generating an output signal to indicate if there is incorrect fitting.

4. The method of any one of claims 1 to 3, further comprising (52) detecting an external ear pressure during a calibration phase to determine subject-specific pressure characteristics.

5. The method of any one of claims 1 to 4, wherein deriving a baseline ear pressure over time comprises using an analytical model of the ear canal.

6. The method of any one of claims 1 to 5, further comprising sensing movement of the subject using an inertia monitoring unit, and detecting drowsiness and/or sleep additionally from the sensed movement.

7. A method of controlling a device, comprising:

    detecting drowsiness and/or sleep using the method of any one of claims 1 to 6; and
    controlling the device in dependence on the detection of drowsiness and/or sleep.

8. The method of claim 7, wherein the device comprises the in-ear speaker, and controlling the device comprises (88) adapting the volume of the audio delivered by the in-ear speaker, or the on-off status of the audio delivery, in dependence on the detection of drowsiness and/or sleep.

9. The method of claim 8, further comprising detecting arousal from sleep and controlling the delivery of audio by the in-ear speaker to improve sleep quality.

10. The method of any one of claims 1 to 9, further comprising controlling a valve to vent the space between the ear drum and the in-ear speaker to the ambient surroundings, in dependence on the detection of drowsiness and/or sleep.

11. A computer program comprising computer program code which is adapted, when said program is run on a computer, to implement the method of any one of claims 1 to 10.

12. A drowsiness and/or sleep detection system, comprising:

an in-ear speaker (10) having a pressure sensor (22) for detecting an external ear pressure between the ear drum and the in-ear speaker; and
a processor (24), wherein the processor is adapted to:

receive a sensed external ear pressure from the pressure sensor;
process frequency and amplitude characteristics of audio that is delivered to the in-ear speaker and the corresponding sensed external ear pressure, thereby to derive a baseline ear pressure overtime; and
detect drowsiness and/or sleep from the derived baseline ear pressure.

13. The system of claim 12, wherein the processor is adapted to detect drowsiness and/or sleep by:

detecting yawning events from the baseline ear pressure; and/or
detecting an increase in middle ear pressure associated with sleep from the baseline ear pressure.

14. An audio delivery system comprising the drowsiness and/or sleep detection system of claim 12 or 13, wherein the processor is adapted to adapt the volume of the delivered audio, or the on-off status of the audio delivery, in dependence on the detection of drowsiness and/or sleep.

15. The system of claim 14, further comprising one or more of:

a sensor arrangement for detecting the correct fitting of the in-ear speaker to the ear, wherein the processor is adapted to generate an output signal to indicate if there is incorrect fitting;
an inertia monitoring unit, wherein the processor is adapted to detect drowsiness and/or sleep additionally from the sensed movement.
a valve to vent the space between the ear drum and the in-ear speaker to the ambient surroundings, wherein the processor is adapted to control the valve in dependence on the detection of drowsiness and/or sleep; or
a sensor arrangement for detecting arousal from sleep, wherein the processor is adapted to control the delivery of audio to improve sleep quality in response to detection of arousal from sleep.

FIG. 1

Fig. 3

FIG. 2

Fig. 2

70 — Switch on music through earplugs

72 — Measure pressure in the external ear (P_Measured)

74 — Analyze the music characteristic (frequency and amplitude)

76 — Estimate pressure changes in the exteral ear due to music (P_Music)

80 — Derive baseline pressure in the external ear (P_BL = f(P_Measured, P_Music))

82 — Detect yawning episodes based on pressure changes

84 — Is yawing frequency over a certian threshold?

Yes       No

86 — Indicates drowsiness

89 — Monitor P_BL

90 — Is there gradual increase in P_BL?

No

92 — Indicates sleeping stage

Yes

88 — Decrease music volume or switch it off

FIG. 3

FIG. 4

Europäisches
Patentamt

European
Patent Office

Office européen
des brevets

# EUROPEAN SEARCH REPORT

Application Number

EP 22 15 7370

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | THOM JOSHUA J. ET AL: "Middle ear pressure during sleep and the effects of continuous positive airway pressure", AMERICAN JOURNAL OF OTOLARYNGOLOGY., vol. 36, no. 2, 1 March 2015 (2015-03-01), pages 173-177, XP055935908, US ISSN: 0196-0709, DOI: 10.1016/j.amjoto.2014.10.024 * the whole document * | 1-15 | INV. A61B5/00 |
| A | EP 3 936 046 A1 (KONINKLIJKE PHILIPS NV [NL]) 12 January 2022 (2022-01-12) * paragraphs [0049], [0050] * | 1-15 | |
| A | US 2018/242908 A1 (SAZONOV EDWARD [US] ET AL) 30 August 2018 (2018-08-30) | 1-15 | |

TECHNICAL FIELDS SEARCHED (IPC)

A61B

The present search report has been drawn up for all claims

| Place of search | Date of completion of the search | Examiner |
|---|---|---|
| The Hague | 28 June 2022 | Lommel, André |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 4 230 114 A1

## ANNEX TO THE EUROPEAN SEARCH REPORT
## ON EUROPEAN PATENT APPLICATION NO.

EP 22 15 7370

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2022

| Patent document cited in search report | | Publication date | Patent family member(s) | | Publication date |
|---|---|---|---|---|---|
| EP 3936046 | A1 | 12-01-2022 | EP | 3936046 A1 | 12-01-2022 |
| | | | WO | 2022008629 A1 | 13-01-2022 |
| US 2018242908 | A1 | 30-08-2018 | US | 2018242908 A1 | 30-08-2018 |
| | | | US | 2020337635 A1 | 29-10-2020 |
| | | | US | 2021345959 A1 | 11-11-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012058886 A

- CN 109561219